(19) **Europäisches Patentamt European Patent Office Office européen des brevets**

(11) **EP 3 360 469 B1**

(12) **EUROPEAN PATENT SPECIFICATION**

(45) Date of publication and mention of the grant of the patent:
**05.05.2021 Bulletin 2021/18**

(21) Application number: **16873240.2**

(22) Date of filing: **14.11.2016**

(51) Int Cl.:
*A61B 5/021* (2006.01)   *A61B 5/024* (2006.01)
*A61B 5/0402* (2006.01)   *A61B 5/0404* (2006.01)
*A61B 5/11* (2006.01)

(86) International application number:
**PCT/KR2016/013057**

(87) International publication number:
**WO 2017/099374 (15.06.2017 Gazette 2017/24)**

(54) **APPARATUS FOR MEASURING BLOOD PRESSURE, AND METHOD FOR MEASURING BLOOD PRESSURE BY USING SAME**

VORRICHTUNG ZUR BLUTDRUCKMESSUNG UND VERFAHREN ZUR BLUTDRUCKMESSUNG DAMIT

APPAREIL DE MESURE DE LA TENSION ARTÉRIELLE, ET PROCÉDÉ DE MESURE DE LA TENSION ARTÉRIELLE L'UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2015   KR 20150173149**
**20.09.2016   KR 20160120252**

(43) Date of publication of application:
**15.08.2018   Bulletin 2018/33**

(73) Proprietor: **Samsung Electronics Co., Ltd.**
**Suwon-si, Gyeonggi-do 16677 (KR)**

(72) Inventors:
• **CHOI, Byung-hun**
  **Suwon-si**
  **Gyeonggi-do 16222 (KR)**
• **KOH, Hyun-woo**
  **Yongin-si**
  **Gyeonggi-do 17077 (KR)**
• **KIM, Hyun-su**
  **Seoul 04375 (KR)**
• **BAEK, Hyun-jae**
  **Seoul 05555 (KR)**
• **SHIN, Jae-wook**
  **Suwon-si**
  **Gyeonggi-do 16532 (KR)**
• **YOON, Chi-yul**
  **Hwaseong-si**
  **Gyeonggi-do 18444 (KR)**

• **CHO, Jae-geol**
  **Yongin-si**
  **Gyeonggi-do 16923 (KR)**

(74) Representative: **Gulde & Partner**
**Patent- und Rechtsanwaltskanzlei mbB**
**Wallstraße 58/59**
**10179 Berlin (DE)**

(56) References cited:
**KR-A- 20080 069 859      KR-A- 20090 052 442**
**KR-A- 20100 116 880      KR-A- 20110 000 287**
**US-A1- 2009 018 422      US-A1- 2010 049 059**
**US-A1- 2010 113 947      US-A1- 2010 241 011**
**US-A1- 2011 009 718      US-A1- 2015 157 220**

• **SEDDIGHEH BAKTASH: "Ratio-Independent Arterial Stiffness-Based Blood Pressure Estimation", UO RESEARCH - UNIVERSITY OF OTTAWA, 1 January 2014 (2014-01-01), pages I-XIV, 1-84, XP055489112, DOI: 10.20381/ruor-5024**

• THOMAS SIMI SUSAN ET AL: "BioWatch: A Noninvasive Wrist-Based Blood Pressure Monitor That Incorporates Training Techniques for Posture and Subject Variability", IEEE JOURNAL OF BIOMEDICAL AND HEALTH INFORMATICS, IEEE, PISCATAWAY, NJ, USA, vol. 20, no. 5, 20 July 2015 (2015-07-20), pages 1291-1300, XP011621636, ISSN: 2168-2194, DOI: 10.1109/JBHI.2015.2458779 [retrieved on 2016-09-01]

**Description**

## BACKGROUND OF THE INVENTION

Field of the Invention

Apparatuses and methods consistent with the present disclosure relate to an apparatus for measuring blood pressure and a method of measuring blood pressure using the same, and more particularly, to an apparatus for measuring blood pressure and a method of measuring blood pressure using the same for detecting, from the wrist, a periodic change occurring when blood flows in a blood vessel via cardiac impulse to measure blood pressure of radial artery.

Description of the Related Art

In general, a non-invasive method that does not damage a human body is a method of measuring blood pressure in a hospital and a home. The non-invasive method is broadly classified into a Korotkoff-sound method and an oscillometric method.

In the Korotkoff-sound method, blood pressure is measured by listening to sound generated in a blood vessel through a stethoscope while increasing and reducing pressure in a cuff in a state in which the cuff is wound around an upper arm and the stethoscope is positioned adjacent to the blood vessel. Systolic blood pressure corresponds to cuff pressure at a time point when stethoscope sound is begun to be first heard (or a time point when two or more pulses of stethoscope sound are begun to be heard). Diastolic blood pressure corresponds to cuff pressure at a time point when stethoscope sound completely disappears by continuously reducing cuff pressure.

However, in the Korotkoff-sound method, a measurer measures blood pressure via stethoscope sound heard through a stethoscope and, thus, the measurer needs high proficiency to accurately measure blood pressure. In addition, because a sense in terms of a time point when stethoscope sound is heard is difference for each measurer, different blood pressure is disadvantageously measured for each measurer.

The oscillometric method is a method of measuring blood pressure using an automated machine and has been most widely used, recently. In the oscillometric method, a cuff is wound around an upper arm and pressure in the cuff is continuously measured while cuff pressure is increased and, then, is gradually reduced. When the pressure in the cuff is continuously measured, a periodic pressure change of blood in a blood vessel is transmitted to the cuff and, in this case, this pressure change in blood changes cuff pressure. In this case, the systolic blood pressure and the diastolic blood pressure are measured by estimating that cuff pressure at a time point when a cuff pressure change due to a blood pressure change is highest is equal to average blood pressure of a user.

However, in the oscillometric method, high pressure needs to be applied to a cuff and, thus, a blood vessel or textus is damaged due to repeated measurement in the case of a hypertensive patient or the old and the inform with inflexible textus. In addition, currently, numerous devices have been introduced but most devices have a large volume and, thus, a user has a difficulty in measuring blood pressure using such a device while carrying the device.

The above two methods of measuring blood pressure commonly use a cuff. Conversely, there is a method of measuring blood pressure using a light sensor instead of a cuff. In the blood pressure measuring method, a pulse transit time (PTT) is calculated using electrocardiogram and photo-plethysmograph (PPG) measured at a finger and blood pressure is estimated using the calculated value. Here, the PPG indicates a pulsation component that is used as a bio signal obtained by irradiating the human body with light in a specific wavelength band and detecting reflected or transmitted light and is generated due to a cardiac impulse. Bio signal measurement using PPG is a non-restraint and non-invasive method and it is possible to detect a multiple signal such as pulse, respiration, and oxygen saturation using a single sensor. A time interval of detecting a pulse wave of PPG from an electronic signal of electrocardiogram (ECG) is referred to as PTT and has a correlation with blood pressure. As such, the method using the PTT measures blood pressure without using of a separate cuff and simultaneously measures PPG and electrocardiogram and calculates the PTT to estimate blood pressure.

However, the PTT is changed by not only a change in blood pressure but also other causes and, thus, there is a problem in that a high measurement error occurs depending on a state of a measurer compared with the above methods using a cuff.

US 2009/0018422 A1 relates to a vital sign monitor for cufflessly measuring blood pressure using a pulse transit time corrected for vascular index and discloses measuring a patient's blood by: 1) measuring a time-dependent optical waveform with an optical sensor; 2) measuring a time-dependent electrical signal with an electrical sensor; 3) estimating the patient's arterial properties using the optical waveform; 4) determining a pulse transit time (PTT) from the time-dependent electrical signal and the time-dependent optical waveform; and 5) calculating a blood pressure value using a mathematical model that includes the PTT and the patient's arterial properties.

US 2010/0241011 A1 relates to the calibration of pulse transit time measurements to arterial blood pressure using external arterial pressure applied along the pulse transit path, wherein a method applies a distributed model with lumped parameters, and uses pulse transit time measurements derived from a wearable photoplethysmograph (PPG) sensor architecture with an intervening pressurizing mechanism.

US 2011/0009718 A1 relates to the determination of physiological parameters using repeated blood pressure measurements, wherein a pulsewave parameters determination unit receives respective first and second signals from pulsewave detection units which generate signals, the signals being responsive to arterial pressure of the portion of the subject's body while the portion of the pulsewave detection unit that is coupled to the portion of the subject's body is at respective first and second heights, at respective times.

## SUMMARY OF THE INVENTION

The present disclosure provides a blood pressure measuring apparatus that is small sized to be worn on the wrist and measures blood pressure in consideration of both a pulse transit time (PTT) and an arterial stiffness index (ASI) that are calculated through photo-plethysmograph (PPG) and electrocardiogram (ECG) to more accurately measure blood pressure, and a blood pressure measuring method using the blood pressure measuring method

According to an embodiment of the present disclosure, a blood pressure measuring method includes calculating, by a controller, a pulse transit time (PTT) using a photo-plethysmograph (PPG) signal and an electrocardiogram (ECG) signal measured by a sensor; calculating, by the controller, an arterial stiffness index (ASI); and calculating, by a controller, systolic blood pressure and diastolic blood pressure using the PTT and the ASI, wherein the ASI is calculated in each of at least two or more postures with different hand shapes by measuring, by the sensor, reference pressure applied to a wrist and fluctuation pressure according to cardiac impulse under the reference pressure with respect to each hand shape, obtaining, by the controller, a graph satisfying values corresponding to the reference pressure and the fluctuation pressure, and calculating, by the controller, a slope through two respectively consecutive points on the graph for which the reference pressure and the fluctuation pressure have been measured.

The hand shapes may be taken by any one of spreading all fingers or folding at least one finger, closing a fist or spreading at least one finger while the fist is closed, by folding a finger, allowing at least two fingers to contact each other, and spacing all fingers apart from each other. In this case, the hand shape may be taken by bending the wrist in any one direction.

The hand shape may be taken by bending the wrist in any one direction and may be taken by bending the wrist at a predetermined angle.

According to an example of the present disclosure, a blood pressure measuring method includes measuring calibration systolic blood pressure and calibration diastolic blood pressure, calculating a pulse transit time (PTT) and an arterial stiffness index (ASI) in a state in which at least two different hand shapes are taken, and calculating the calibration systolic blood pressure and calibration diastolic blood pressure, calibration pulse transit time and calibration ASI, and systolic blood pressure and diastolic blood pressure through the calculated PTT and ASI.

The PTT is calculated using the PPG signal and the electrocardiogram signal.

While not falling under the scope of the invention, the ASI may be calculated according to a following math formula by measuring reference pressure applied to a wrist and fluctuation pressure according to cardiac impulse under the reference pressure with respect to each hand shape and obtaining a graph satisfying values corresponding to the reference pressure and the fluctuation pressure:

$$y = a \cdot e^{bx} + c$$

where x is the reference pressure, y is the fluctuation pressure, b is the ASI, and 'a' and 'c' are constants.

The diastolic blood pressure may be calculated according to following math formulae:

$$P_{SD0} = P_{S0} - P_{D0}$$

where $P_{S0}$ is calibration systolic blood pressure and $P_{D0}$ is calibration diastolic blood pressure.

$$P_{SD} = P_{SD0} \left( \frac{e^b}{e^{b_c}} \right) \left( \frac{PTT_0}{PTT} \right)^2$$

where $PTT_0$ is a calibration pulse transit time, PTT is a pulse transit time during blood pressure measurement, $b_0$ is a calibration ASI, and b is an ASI during blood pressure measurement.

$$P_D = \frac{e^b (b_0 - b)}{0.018} + \frac{e^b}{e^{b_0}} (P_{D0} + k_0 P_{SD0}) - k P_{SD}$$

where $P_D$ is diastolic blood pressure during blood pressure measurement and *k* is a constant.

The systolic blood pressure may be obtained according to a following math formula:

$$P_S = P_D + P_{SD}$$

where $P_S$ is systolic blood pressure during blood pressure measurement.

The k may be calculated according to a following math formula:

$$k = \frac{\int_a^b f(x)}{|Bb - Ba| \cdot H}$$

where f(x) is an area of a waveform configured by fluctuation pressure, Bb-Ba is a width of a fluctuation waveform, and H is a height of the fluctuation waveform.

The blood pressure measuring method may further include guiding a first hand shape, measuring a first pressure deviation applied to a wrist for a predetermined time, re-guiding the first hand shape when the first pressure deviation is not within a predetermined range and measuring a first ASI when the first pressure deviation is within the predetermined range, guiding a second hand shape, determining whether a first inclination of pressure applied to the wrist is greater than a second inclination of pre-measured pressure applied to the wrist as a hand shape is changed to the second hand shape from the first hand shape, re-guiding the second hand shape when the first inclination is less than the second inclination and measuring a second pressure deviation applied to the wrist for a predetermined time when the first inclination is greater than the second inclination, and re-guiding the second hand shape when the second pressure deviation is not within a predetermined range and measuring a second ASI when the second pressure deviation is within the predetermined range.

The first and second hand shapes may be guided to at least one of an image, a text, and voice.

The measuring of the ASI may include guiding a predetermined wearing posture of a blood pressuring measuring apparatus according to whether an ECG signal is detected.

According to another embodiment of the present disclosure, a blood pressure measuring apparatus includes a wearing portion configured to be worn on a wrist of a user; a sensor disposed on the wearing portion and configured to measure a photo-plethysmograph (PPG) signal and an electrocardiogram (ECG) signal, a controller configured to calculate a pulse transit time (PTT) using the photo-plethysmograph (PPG) signal and the electrocardiogram (ECG) signal, and an arterial stiffness index (ASI) of the user and calculate systolic blood pressure and diastolic blood pressure of the user using the PTT and the ASI and a display configured to display the systolic blood pressure and diastolic blood pressure calculated by the controller, wherein the sensor is further configured to detect pressure in a state in which at least two different hand shapes are taken to vary a degree pressurized to the wrist by the wearing portion, wherein the controller is further configured to calculate the ASI by measuring, by the sensor, reference pressure applied to a wrist and fluctuation pressure according to cardiac impulse under the reference pressure with respect to each hand shape, obtain a graph satisfying values corresponding to the reference pressure and the fluctuation pressure, and calculate a slope through two respectively consecutive points on the graph for which the reference pressure and the fluctuation pressure have been measured.

The sensor may include a PPG sensor, an electrocardiogram sensor, and a pressure sensor.

The sensor may detect reference pressure applied to a wrist of a user and fluctuation pressure varied according to cardiac impulse under the reference pressure as pressure of a cell, which is installed on the wearing posture and into which air is injected, to pressurize the cell according to a hand shape.

The sensor may be disposed on one surface of the wearing portion to directly detect pressure applied to a wrist of a user according to a hand shape.

The sensor may be disposed between the wearing portion and the wrist

The invention is defined by an apparatus according to claim 11 and a method according to claim 1.

## BRIEF DESCRIPTION OF THE DRAWING FIGURES

FIG. 1 is a schematic block diagram a blood pressure measuring apparatus according to an exemplary embodiment of the present disclosure.

FIG. 2A is an exploded perspective view showing a blood pressure measuring apparatus according to an exemplary embodiment of the present disclosure.

FIG. 2B is a coupling perspective view showing a blood pressure measuring apparatus according to an exemplary embodiment of the present disclosure.

FIG. 3 is a perspective view showing an example in which a position of a pressure sensor is changed.

FIG. 4 is a perspective view showing a state in which a blood pressure measuring apparatus is worn on the wrist to measure blood pressure according to an exemplary embodiment of the present disclosure.

FIG. 5 is a flowchart of a method of measuring blood pressure according to an exemplary embodiment of the present disclosure.

FIG. 6 is a graph showing an output waveform of photo-plethysmograph (PPG) and electrocardiogram (ECG).

FIG. 7A is a graph showing pressure with respect to different hand shapes for detection of an arterial stiffness index (ASI).

FIG. 7B is a schematic diagram showing a hand shape with a varied angle of bending the wrist.

FIG. 8 is an enlarged vie of a portion indicated in FIG. 7A.

FIG. 9 is a graph showing an ASI obtained via reference pressure and fluctuation pressure corresponding to at least two different hand shapes.

FIG. 10 is a graph showing a ratio of an area occupied by a pressure waveform of a partial period taken from the pressure waveform shown in FIG. 8.

FIG. 11 is a flowchart showing a procedure of measuring blood pressure while guiding a user to take first and second hand shapes.

FIG. 12 is a flowchart showing a procedure of determining whether a wearing portion is appropriately worn on the wrist through an ECG signal.

FIG. 13A is a diagram showing a state in which a second electrocardiogram electrode of an ECG sensor is spaced apart from the wrist and FIG. 13B is a diagram showing a state in which a second electrocardiogram electrode of an ECG sensor contacts the wrist.

## DETAILED DESCRIPTION OF THE EXEMPLARY EMBODIMENTS

The terms, such as "first", "second", and the like used in this disclosure may be used to refer to various elements regardless of the order and/or the priority and to distinguish the relevant elements from other elements, but do not limit the elements. For example, "a first user device" and "a second user device" indicate different user devices regardless of the order or priority. For example, without departing the scope of the present disclosure, a first element may be referred to as a second element, and similarly, a second element may be referred to as a first element.

The term blood pressure stated in this specification refers to a pressure value exerted on a blood vessel wall when blood transmitted from the heart flows in a blood vessel and is classified into arterial pressure, capillary blood pressure, venous pressure, and so on according to a name of the blood vessel. Blood pressure is changed by heart beat. In addition, blood pressure includes systolic blood pressure and diastolic blood pressure. Systolic blood pressure is blood pressure when a ventricle contracts and blood is pushed into artery. Diastolic blood pressure is blood pressure when blood is pressed due to stiffness of an arterial wall even if a ventricle is enlarged and blood is not pushed out.

A sphygmus wave is a wave formed while a pulse is transmitted up to peripheral nerves. The pulse is a phenomenon whereby pressure in a blood flow introduced into an aorta appears in other arteries due to a cardiac impulse. That is, whenever the heart contracts, blood is supplied to a whole body from the heart through an aorta and pressure in the aorta is changed. This hange in pressure in the aorta is transmitted to peripheral arterioles of the hand and foot. The sphygmus wave corresponds to this change in pressure represented in the form of a waveform.

The blood pressure apparatus according to the present disclosure may belong to a wearable device that is worn around the wrist and may be capable of be carried by a user to simply measure blood pressure anywhere. In particular, the apparatus may detect a pulse transit time (PTT) and an arterial stiffness index (ASI) and may accurately measure blood pressure using the detected values. In this case, to measure an ASI, different hand shapes may be formed according to various shape gestures and different pressures (default pressure) corresponding to the respective hand shapes may be detected. Under this pressure, pressure (fluctuation pressure) that is continuously changed according to heart beat may be detected. As such, under different default pressures for the respective hand shapes, the ASI may be detected via fluctuation pressure indicated according to a cardiac impulse. As described above, the PTT and the ASI may be calculated to calculate accurate blood pressure using the detected values. A detailed method of calculating blood pressure is described below.

Here, the various hand shapes may be, for example, a hand shape that is changed via an action of spreading out the fingers one by one from a state in which the fist is closed or a hand shape taken via an action of changing an angle of bending the wrist from a state in which the fist is closed or open. These various hand shapes may be used to apply different default pressures to the radial artery. Any hand shape that is not stated in the present exemplary embodiment may also be applied as long as pressure applied to the radial artery is basically changed. The radial artery may be an artery that is branched from an upper arm artery, is reached up to the palm through an edge portion of a bottom arm, and is positioned between the radius and the skin in the wrist part and may be a portion on which the pulse is mainly felt.

Hereinafter, a configuration of a blood pressure measuring apparatus according to an exemplary embodiment of the present disclosure is described in detail and, then, a method of measuring blood pressure using the blood pressure measuring apparatus is described in detail.

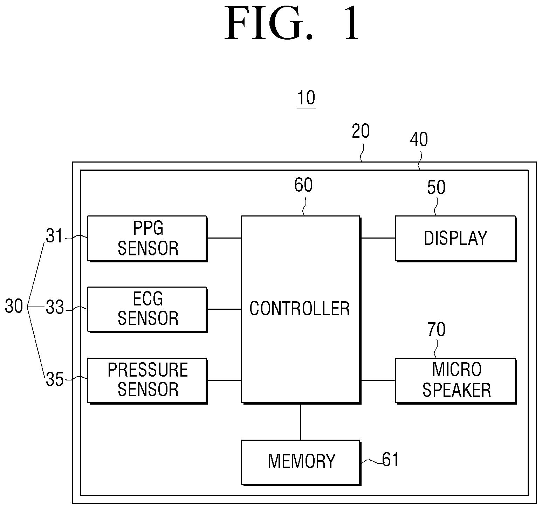

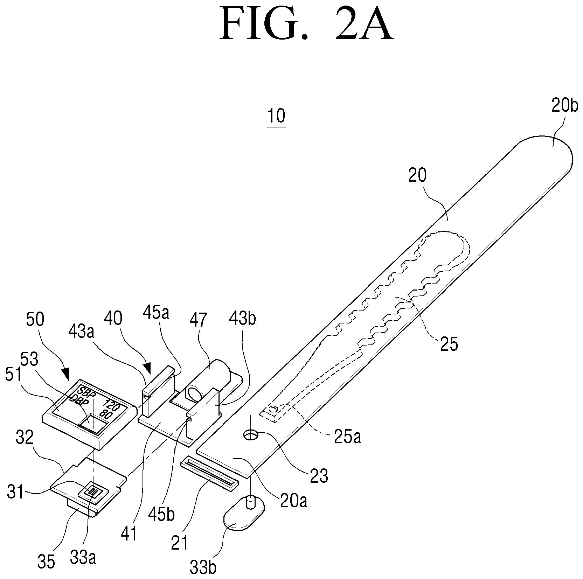

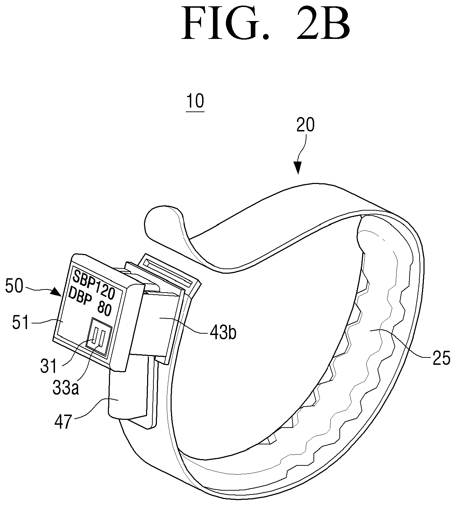

FIG. 1 is a schematic block diagram a blood pressure measuring apparatus according to an exemplary embodiment of the present disclosure. FIGS. 2A and 2B are an exploded perspective view and a coupling perspective view showing a blood pressure measuring apparatus, respectively according to an exemplary embodiment of the present disclosure.

Referring to FIG. 1, a blood pressure measuring apparatus 10 may include a wearing portion 20 worn on the wrist, a sensor 30 for detecting a pressure value and sphygmus wave applied to a blood vessel present in a part at which blood pressure is measured, a support portion 40 coupled to the wearing portion 20 to support the sensor 30, and a display 50 coupled to the support portion 40 to display blood pressure calculated through the pressure value and the sphygmus wave that are detected by the sensor 30. In the specification, to prevent the features of the present exemplary embodiment from being obscure, only hardware components related to the present exemplary embodiment are described. It would be obvious to one of ordinary skill in the art to which the present exemplary embodiment pertains that the blood pressure measuring apparatus includes generic-purpose components other than the hardware components illustrated in FIG. 1.

Referring to FIG. 2A, the wearing portion 20 may have a predetermined length to allow the wearing portion 20 to be worn on the user wrist. When the user takes a predetermined hand shape in a state in which the wearing portion 20 is worn on the user wrist, the radial artery may be pressurized by the wearing portion 20. A one end portion 20a of the wearing portion 20 may include a buckle 21 to maintain the state in which the wearing portion 20 is worn on the wrist. The wearing portion 20 may be worn on the wrist by inserting the other end portion 20b of the wearing portion 20 into the buckle 21 while winding the user wrist. On the other hand, when the other end portion 20b of the wearing portion 20 is separated from the buckle 21, wearing of the wearing portion 20 may be released from the wrist.

A second electrocardiogram electrode 33b that is disposed adjacent to the buckle 21 to detect electrocardiogram may be arranged on the wearing portion 20. The wearing portion 20 may include a through hole 23 to which a portion of the second electrocardiogram electrode 33b is coupled and which is formed in a corresponding portion on which the second electrocardiogram electrode 33b is arranged. The second electrocardiogram electrode 33b may be fixed to the wearing portion 20 as the portion of the second electrocardiogram electrode 33b is coupled to the through hole 23.

A cell 25 into which air is injected may be formed in the wearing portion 20 along a longitudinal length thereof. The cell 25 may contact a portion of the user wrist in a state in which the wearing portion 20 is worn the wrist. When the user changes the hand shape to measure blood pressure, the cell 25 may be pressurized by an external surface of the wrist as the thickness of the wrist is changed. The thickness of the wrist may be changed as the wrist muscle contracts or is relaxed when the user takes a predetermined hand shape. For example, the thickness of the wrist in a state in which all fingers are spread may be larger than that of the wrist in a state in which the fist is closed. As the wrist is changed, pressure applied to the cell 25 may also be changed. The changed pressure of the cell 25 may be detected by a pressure sensor 35.

The sensor 30 may detect a pressure value and sphygmus wave applied to the blood vessel. Here, the blood vessel refers to a blood vessel inside a part that is pressurized while pressure is applied to the part as a measurement target part of blood pressure in a state in which the user maintains a predetermined hand shape.

The sensor 30 may include a photo-plethysmograph (PPG) sensor 31 and an electrocardiogram (ECG) sensor 33 for calculation of a pulse transit time (PTT). The sensor 30 may also include the pressure sensor 35 for calculation of pressure of radial artery depending on a hand shape to measure the arterial stiffness index (ASI).

The PPG sensor 31 may be a sensor for analyzing a signal obtained by emitting light beams with two or more different wavelengths to a user finger that contacts a first electrocardiogram electrode 33a of the ECG sensor 33 and receiving the light beams. In this case, the PPG sensor 31 may also be used as an oxygen saturation sensor and, in this regard, the oxygen saturation may be mainly used to calculate calorie consumption according to user activity and may also be used to monitor a situation such as labored respiration, clouded consciousness, shock, body condition during exercise, adult respiratory distress syndrome (ARDS) that is a lung disease, hypoxia risk detection in an alpine region, gas poisoning, and a choking incident. The PPG sensor 31 may be arranged adjacent to the first electrocardiogram electrode 33a of the ECG sensor 33, as shown in FIG. 2A.

The ECG sensor 33 may also be referred to as an electrocardiography (EKG) sensor and may be a sensor for detection of a pattern signal of action current of the heart. The electrocardiogram sensor may be classified into a current electrocardiograph and a voltage electrocardiograph according to a signal detection method. The electrocardiogram waveform may include P, Q, R, S, and T waves. Thereamong, the R wave may correspond to a peak signal and, thus, may be mainly used to analyze a bio signal. For example, a heart rate may be measured through the number of R-wave signals generated per unit time and may be used to diagnose arrhythmia such as tachycardia or bradycardia and to measure overall heart performance ability.

The ECG sensor 33 may include the two electrocardiogram electrodes 33a and 33b to measure electrocardiogram. The first electrocardiogram electrode 33a may be disposed on the other surface of a printed circuit board 32 and may be arranged adjacent to the PPG sensor 31. Accordingly, when a user measures blood pressure, a finger F of the other hand R on which the blood pressure measuring apparatus 10 is not worn may simultaneously contact the first electrocardiogram electrode 33a and the PPG sensor 31 of the ECG sensor 33. The second electrocardiogram electrode 33b may be arranged on one surface of the wearing portion 20 to contact an upper portion of the wrist when the wearing portion 20 is worn on the wrist. In this case, the second electrocardiogram electrode 33b may be maintained to be electrically connected to the ECG sensor 33 installed on the printed circuit board 32. The ECG sensor 33 may detect a pattern signal of action current of the heart using a voltage difference between the first and second electrocardiogram electrodes 33a and 33b.

The pressure sensor 35 may measure default pressure applied to the radial artery by the wearing portion 20 and fluctuation pressure varied according to a cardiac impulse with respect to a predetermined hand shape when the user takes the predetermined hand shape. In this case, the pressure sensor 35 may be connected to the cell 25 formed in the wearing portion 20 through a connection tube 47. Accordingly, the pressure sensor 35 may indirectly detect the default pressure and the fluctuation pressure through pressure of the cell 25 and a change in the pressure.

The support portion 40 may be used as a connection element for coupling the sensor 30 to the wearing portion 20. The support portion 40 may include a coupling piece 41 fixed to one surface of the wearing portion 20, a pair of protrusion pieces 43a and 43b that protrude in parallel to each other to be spaced apart from one surface of the coupling piece 41 at a predetermined interval, and the connection tube 47.

The coupling piece 41 may be fixed to the wearing portion 20 via adhesives or thermosetting or, needless to say, may be fixed to the wearing portion 20 through a general coupling hole such as a screw.

The pair of protrusion pieces 43a and 43b may include slots 45a and 45b formed therein to slidably couple opposite ends of the printed circuit board 32 to the slots 45a and 45b, respectively. In addition, the display 50 may be coupled to upper ends of the pair of protrusion pieces 43a and 43b.

The connection tube 47 may be connected to a connection hole 25a formed in the cell 25 through a predetermined connection pipe (not shown) and, thus, may be indirectly connected to the pressure sensor 35.

The display 50 may include a display region 51 disposed on one surface thereof to display UI/UX. The display region 51 may display the measured systolic blood pressure (SBP) and diastolic blood pressure (DBP) or display a guidance message for blood pressure measurement, that is, a message indicating finger F contact with an electrode, a message indicating that at least two hand shapes need to be maintained for respective predetermined time periods (several seconds), and a message indicating that blood pressure measurement is completed.

When a current posture is not an appropriate posture (hand shape) for blood pressure measurement, the display 50 may display a notice indicating this.

The display 50 may display various pieces of information such as current time when blood pressure is not measured.

The display 50 may include a touchscreen and display UI/UX for input of information as well as information to receive data directly from the user. For example, in some embodiments, when blood pressure is measured, user blood pressure measured by a general blood pressure measurer needs to be input at least one time and, in this case, the display 50 may receive the user blood pressure through the UI/UX displayed on the display region 51.

The display 50 may include an exposure hole 53 that is formed in one side thereof and through which the PPG sensor 31 and the first electrocardiogram electrode 33a of the ECG sensor 33 are exposed. In this case, the exposure hole 53 may be arranged on the same surface as one surface of the display 50, on which the display region 51 is formed.

A controller 60 may receive signals detected from the sensor 30 and calculate blood pressure through the signals. The controller 60 may include a microprocessor installed on the printed circuit board 32. The controller 60 may be electrically connected to a memory 61 that temporarily stores the detected signal and the memory 61 may store a program a program for calculate the detected signal. The program may calculate a PTT using user systolic blood pressure and diastolic blood pressure measured through a separate blood pressure measuring device and a PPG detection value and ECG detection value detected by the sensor 30 and may also be programmed to execute the following math formulae for calculating an arterial stiffness index (ASI) to calculate systolic blood pressure and diastolic blood pressure according to blood pressure measurement of the user.

The controller 60 may control the display 50 to display the user systolic blood pressure and diastolic blood pressure obtained using the above program on the display region 51.

Although, according to the present exemplary embodiment, blood pressure information is displayed through the display 50 included in the blood pressure measuring apparatus 10, the present exemplary embodiment is not limited thereto and, thus, the measured blood pressure information may be transmitted to a separate device, e.g., a mobile device or immobile device including a display and displayed on the display of the corresponding device. In this case, the blood pressure measuring apparatus 10 may transmit the blood pressure information to the corresponding device and the corresponding device may receive the blood pressure information. As such, to transmit and receive the blood pressure information, the blood pressure measuring apparatus 10 and the corresponding device may each include a wireless mobile module (e.g., a wireless mobile module using Bluetooth, Wi-Fi, ZigBee, or the like).

The blood pressure measuring apparatus 10 according to the present exemplary embodiment of the present disclosure may further include a micro speaker 70 for output of guidance voice or guidance sound to allow a user to further move the hand or to take another hand shape when a hand shape taken to measure an ASI does not apply sufficient pressure to a blood vessel. Needless to say, a character or image corresponding to the guidance voice may be displayed through the display 50 of the blood pressure measuring apparatus 10. When the blood pressure measuring apparatus 10 is connected to a mobile device, the guidance voice or the guidance sound may be output through a speaker installed in the mobile device and the character or image corresponding to the guidance voice may be displayed through the display of the mobile device.

The blood pressure measuring apparatus 10 according to the present exemplary embodiment may predetermine time for blood pressure measurement and, when the predetermined time elapses during blood pressure measurement, sound, character, or image information indicating that measurement is completed to the user may be displayed through the display 50 or the micro speaker 70.

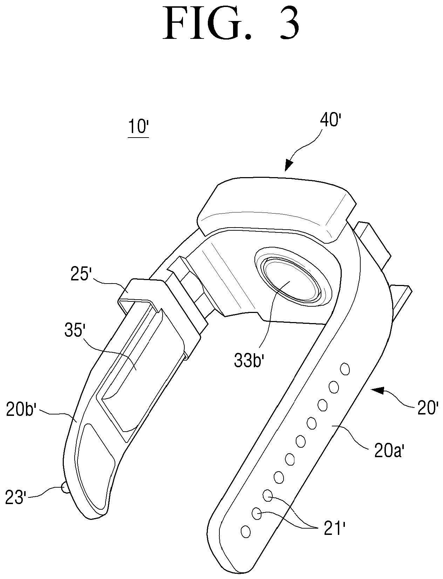

FIG. 3 is a perspective view showing an example in which a position of a pressure sensor is changed.

Referring to FIG. 3, a blood pressure measuring apparatus 10' may include components, most of which are the same as those of the aforementioned blood pressure measuring apparatus 10. However, a position of a pressure sensor 35' may be set to one surface of a wearing portion 20'. In this case, the pressure sensor 35' may directly contact the wrist when the wearing portion 20' is worn on the wrist and, when the user takes a predetermined hand shape to measure blood pressure, pressure applied to the wrist (i.e., the radial artery of the wrist) may be directly detected without the cell 25. Accordingly, the cell 25 and the connection tube 47 connected to the cell 25 may be omitted.

The pressure sensor 35' may be electrically connected to the controller 60 to transmit the detected pressure signal to the controller 60. A second electrocardiogram electrode 33b' of an ECG sensor may be arranged on one surface of the wearing portion 20', on which the pressure sensor 35' is disposed, to contact an upper portion of the wrist.

In the blood pressure measuring apparatus 10', a configuration of the wearing portion 20' may be slightly different from the aforementioned blood pressure measuring apparatus 10'. That is, the wearing portion 20' may include a plurality of coupling holes 21' that are formed in one side portion 20a' with respect to a portion, to which a support portion 40' is coupled, to be spaced apart from each other at a predetermined interval in a longitudinal direction of the wearing portion 20', and a coupling protrusion 23' formed on the other side portion 20b' to be coupled to any one of the plurality of coupling holes 21'. In this case, a buckle 25' to which the one side portion 20a' is coupled may be arranged on the other side portion 20b' of the wearing portion 20'.

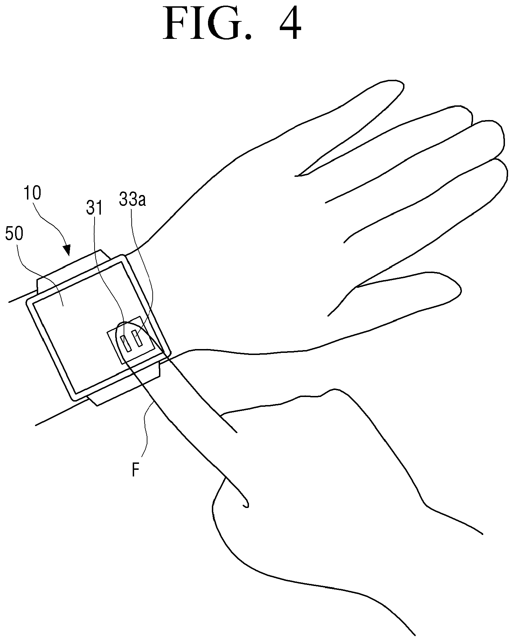

Hereinafter, a procedure of detecting blood pressure using the blood pressure measuring apparatus 10 according to the present disclosure is sequentially described with reference to FIGS. 4 to 10.

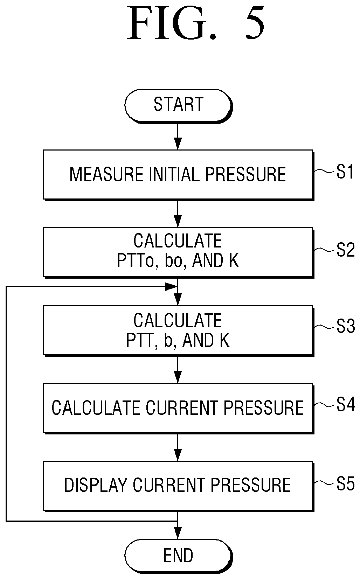

FIG. 4 is a perspective view showing a state in which a blood pressure measuring apparatus is worn on the wrist to measure blood pressure according to an exemplary embodiment of the present disclosure. FIG. 5 is a flowchart of a method of measuring blood pressure according to an exemplary embodiment of the present disclosure.

Referring to FIG. 4, in the blood pressure measuring method according to the present exemplary embodiment, as a first operation, user calibration systolic blood pressure PSO and calibration diastolic blood pressure PD0 measured through a general blood pressure measurer (S1). As shown in FIG. 5, in a state in which the blood pressure measuring apparatus 10 is worn the wrist, a calibration pulse transit time value $PTT_0$, a calibration ASI value $b_0$, and a calibration k value $k_0$ may be calculated through the blood pressure measuring apparatus 10 (S2).

Then, when blood pressure is measured through the blood pressure measuring apparatus 10 according to the present exemplary embodiment, a pulse transit time value PTT, an ASI value b, and a *k* value *k* may be calculated through the blood pressure measuring apparatus 10 without necessity of a separate general blood pressure measurer (S3) and systolic blood pressure PS and diastolic blood pressure PD may be (in an example not falling under the scope of the claims) calculated accordng to Math Formulae 1 to 4 below using the pre-calculated calibration values (the calibration ASI value $b_0$, the calibration pulse transit time value $PTT_0$, and the calibration *k* value $k_0$) (S4). The calculated systolic blood pressure PS and diastolic blood pressure PD may be displayed on the display region 51 (refer to FIG. 2B) of the display 50.

Operations S1 and S2 are performed only in the first operation in the blood pressure measuring method according to the present exemplary embodiment and only operations S3 to S5 may be performed during next measurement of blood pressure.

In operations S2 and S3, the user may take the following action to detect the pulse transit time values $PTT_0$ and PTT and the ASI values $b_0$ and b.

That is, as shown in FIG. 5, the finger F of a hand that does not wear the blood pressure measuring apparatus 10 may simultaneously contact the PPG sensor 31 and the first electrocardiogram electrode 33a of the ECG sensor 33. In this state, the hand that wears the blood pressure measuring apparatus 10 may be moved to maintain first hand shape for a predetermined time period (several seconds) and, continuously, to maintain second hand shape different from the first hand shape for a predetermined time period (several seconds).

Accordingly, a PPG signal (waveform) and an ECG signal (waveform) may be obtained through the PPG sensor 31 and the ECG sensor 33, respectively, in a state in which the first hand shape and the second hand shape are taken. In this case, a waveform corresponding to the PPG waveform may be calculated using the pressure sensor 35 instead of the PPG sensor 31. According to the present exemplary embodiment, default pressure applied to the radial artery and fluctuation pressure varied according to a cardiac impulse may be calculated through the pressure sensor 35 in a state of the first hand shape and the second hand shape. As such, the PPG signal (waveform), the ECG signal (waveform), the default pressure, and the fluctuation pressure may be detected in a state in which at least two different hand shapes are taken, thereby enhancing the reliability of measurement result.

A PTT value may be calculated through the detected PPG signal (waveform) and ECG signal (waveform) and the ASI may be calculated using the default pressure and the fluctuation pressure. In addition, according to the present exemplary embodiment, the prepared calibration systolic blood pressure $P_{SO}$ and diastolic blood pressure $P_{DO}$, the calibration pulse transit time value $PTT_0$, the calibration ASI value $b_0$, and the calibration *k* value $k_0$ with the currently measured pulse transit time value PTT, ASI value b, and *k* value *k* may be inserted into Math Formulae 1 to 4 below to accurately measure the currently measured blood pressure.

## Math Formula 1

$$P_{SD0} = P_{S0} - P_{D0}$$

## Math Formula 2

$$P_{SD} = P_{SD0} \left( \frac{e^b}{e^{b_s}} \right) \left( \frac{PTT_0}{PTT} \right)^2$$

## Math Formula 3

$$P_D = \frac{e^b (b_0 - b)}{0.018} + \frac{e^b}{e^{b_s}} (P_{D0} + k_0 P_{SD0}) - k P_{SD}$$

## Math Formula 4

$$P_S = P_D + P_{SD}$$

Hereinafter, a procedure of calculating $PTT_0$ and PPT values, ASI values $b_0$ and b, $k_0$ and $k$ values that are to be inserted into Math Formulae 2 and 3 above through the sensor 30 is described. The values $PTT_0$ and PPT may be calculated using the same procedure, the values $b_0$ and b may be calculated using the same procedure, and the values $k_0$ and $k$ may be calculated using the same procedure and, thus, hereinafter, only a procedure of calculating the values PPT, b, and $k$ is described.

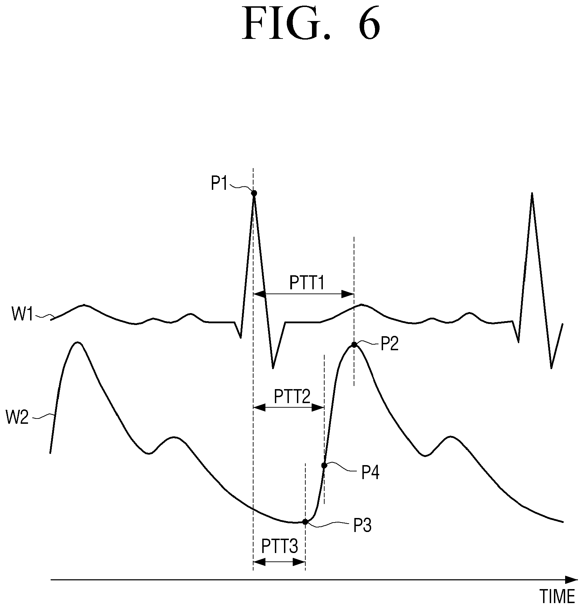

First, a procedure of calculating a value PPT is described with reference to FIG. 6. FIG. 6 is a graph showing an output waveform of photo-plethysmograph (PPG) and electrocardiogram (ECG).

When being discharged from a left ventricle according to a cardiac impulse, a blood flow may be moved along a blood vessel and a periodic flow thereof may also be detected in the radial artery of the wrist. In this case, an output signal measured every heartbeat through the PPG sensor 31 and the ECG sensor 33 may be indicated by a first waveform W1 and a second waveform W2 shown in FIG. 6. The first waveform may be an output signal detected by the PPG sensor 31 and the second waveform may be an output signal detected by the ECG sensor 33.

In this case, PTT[1] indicates an interval between a peak P1 of the first waveform W1 detected by the ECG sensor 33 and a peak P4 of the second waveform W2 detected by the PPG sensor 31, PTT[2] indicates an interval between the peak P1 of the first waveform W1 and a valley P3 of the second waveform W2, and PTT[3] indicates an interval between the peak P1 of the first waveform W1 and a first differentiation maximum value P4 of the second waveform W2. The value PTT to be applied to the Math Formulae 2 and 3 above may use PTT[3] between PTT[1] and PTT[2].

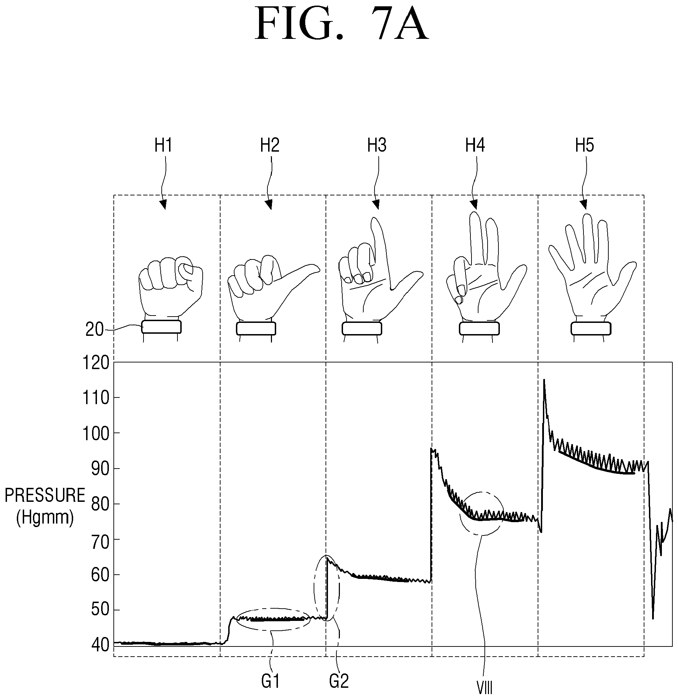

Then, a procedure of calculating a value b that is an ASI is described with reference to FIG. 7A to 9.

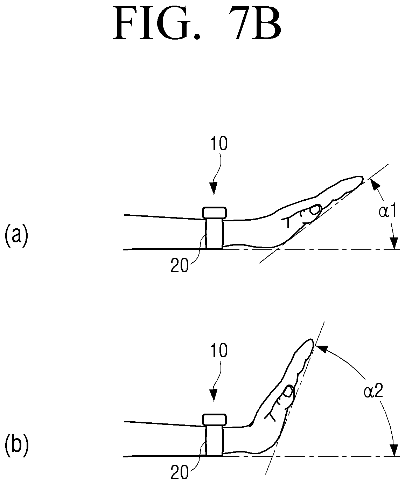

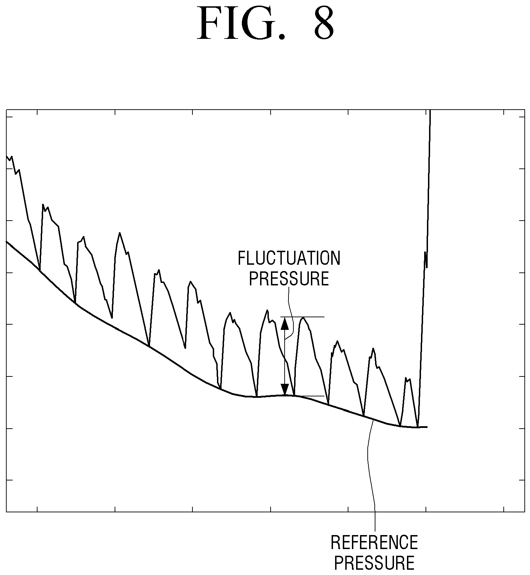

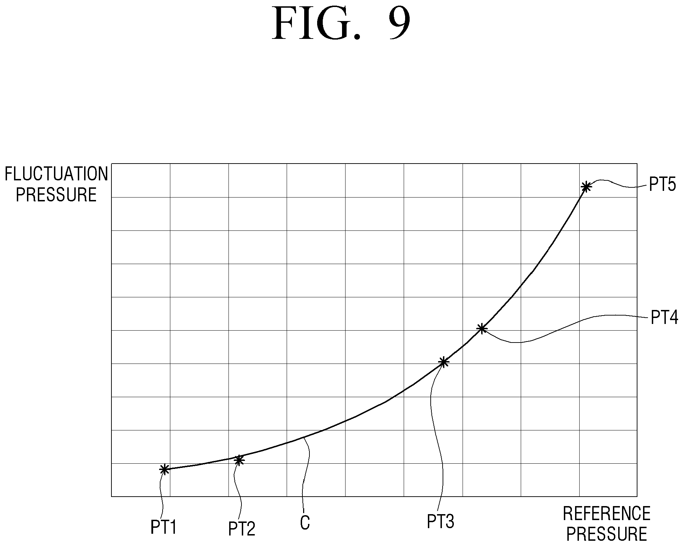

FIG. 7A is a graph showing pressure with respect to different hand shapes for detection of an ASI. FIG. 7B is a schematic diagram showing a hand shape with a varied angle of bending the wrist. FIG. 8 is an enlarged view of a ortion indicated in FIG. 7A. FIG. 9 is a graph showing an ASI obtained via reference pressure and fluctuation pressure corresponding to at least two different hand shapes.

As shown in FIG. 5, when any one of hand shapes is taken by moving fingers in a state in which the blood pressure measuring apparatus 10 according to the present disclosure is worn, pressure measured at the wrist may be changed compared with the case before the hand shape is taken. As such, with regard to a pressure signal measured through the pressure sensor 35 in a state in which any one hand shape is taken, pressure signals in the case of different hand shapes indicate different waveforms and the waveforms reflect a state of a blood vessel, as shown in FIG. 7A.

As shown in FIG. 7A, different hand shapes H1 to H5 indicate different pressures signals and, in this regard, pressure corresponding to hand shapes H2 to H5 in which fingers are spread one by one is increased compared with pressure of the hand shape in which the fist is closed. This is because, when the fist is closed, muscles at the lower arm and the wrist contract and pressure applied to the wrist by the wearing portion 20 is reduced and, thus, whenever fingers are spread one by one, the pressure applied to the wrist by the wearing portion 20 is gradually increased as the muscles are gradually relaxed.

As such, the present exemplary embodiment may provide a condition of applying pressure with different degrees to the wrist according to a hand shape to measure an ASI.

A hand shape may take various hand shapes other than the example illustrated in FIG. 7A. For example, as shown in FIG. 7B, a posture with a varied angle $\alpha 1$ and $\alpha 2$ of bending the wrist may be taken. Although FIG. 7B shows a hand shape in which the wrist is bent upward, the present exemplary embodiment is not limited thereto and, thus, hand shapes in which the wrist is bent at a predetermined angle in a downward, left, right, or diagonal direction may be taken. In this case, a hand shape in which the wrist is bent and a hand shape in which the wrist is not bent may be interchangeably taken.

Although not shown in the drawing, the hand shapes shown in FIGS. 7A and 7B may be simultaneously taken. That is, in a state in which any one of the hand shapes shown in FIG. 7A is maintained, a hand shape in which the wrist is bent at any one angle may be taken, as shown in FIG. 7B.

Although not shown in the drawing, the hand shape may be taken as any one of hand shapes in which fingers are widely spread in such a way fingers do not contact each other, a hand shape in which at least one finger is folded at a predetermined angle in a state in which all fingers are spread, a hand shape in which fingers are folded, a hand shape in which at least two fingers contact each other, and a hand shape in which all fingers do not contact. When these hand shapes are taken, the wrist may be simultaneously bent.

As such, according to the present exemplary embodiment, a hand shape to measure an ASI is not particularly limited and, thus, any hand shape to be taken by a user may be possible.

When a portion of a pressure signal with respect to any one hand shape H4 shown in FIG. 7A is enlarged, the pressure signal may include reference pressure and fluctuation pressure indicating a fluctuation waveform with respect to the reference pressure, as shown in FIG. 8. The reference pressure is pressure applied to the wrist by the wearing portion 20 according to a hand shape and the fluctuation pressure is pressure that continuously fluctuates according to cardiac impulse under the reference pressure. Amplitude of the fluctuation pressure may be determined according to the size of the reference pressure, a state (hardness) of a blood vessel, and blood pressure.

Referring to FIG. 9, an inclination of a graph of an exponential function represented by Math Formula 5 according to fluctuation pressure corresponding to reference pressure may be calculated to calculate an ASI 'b'. According to the present exemplary embodiment, according to the fact indicating that, when an ASI is changed, an inclination of a graph of fluctuation pressure measured using an oscillometric method is changed, the inclination of the graph may be calculated to calculate an ASI via a change in pressure measured when different pressures are applied to the wrist according to a hand shape.

## Math Formula 5

$$y = a \cdot e^{bx}$$

Here, x is a value corresponding to default pressure, y is a value corresponding to fluctuation pressure, and 'a' is a constant.

A plurality of points PT1 to PT5 shown in FIG. 9 may be obtained by default pressure and fluctuation pressure corresponding to the five hand shapes H1 to H5 shown in FIG. 7A and, when the points PT1 to PT5 are connected, an exponential function graph shown in FIG. 9 may be obtained.

According to the present exemplary embodiment, to calculate an ASI, all the five hand shapes are taken and the exponential function graph shown in FIG. 9 is obtained via the default pressure and the elastic pressure obtained under each of the hand shapes but, as seen from the above description, a graph of an ASI is represented as a graph of an exponential function and, thus, to calculate default pressure and fluctuation pressure only for at least two hand shapes, according to the invention, an inclination is calculated through two points to calculate a ASI. Accordingly, a user may measure blood pressure in a state in which at least two different hand shapes are taken.

Math Formula 5 may be represented as an exponential function including a constant 'c' like in Math Formula 6.

## Math Formula 6

$$y = a \cdot e^{bx} + c$$

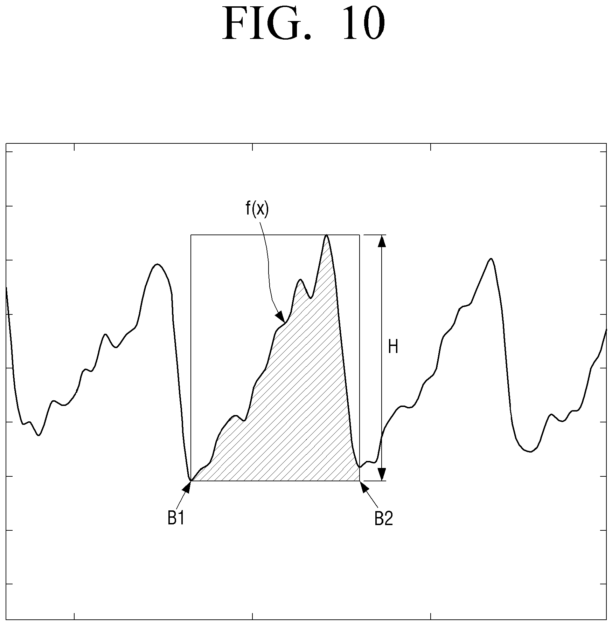

FIG. 10 is a graph showing a ratio of an area occupied by a pressure waveform of a partial period taken from the pressure waveform shown in FIG. 8.

A value k to be applied to Math Formula 3 is a ratio of an area occupied by a fluctuation pressure waveform to an area that is a product of a width Bb-Ba and a height H of the fluctuation pressure waveform as indicated in a math formula 7 below.

## Math Formula 7

$$k = \frac{\int_a^b f(x)}{|Bb-Ba| \cdot H}$$

A value $k$ may be replaced with an appropriate constant to calculate measurement blood pressure rather than being calculated every calibration or measurement time point.

As such, the blood pressure measuring apparatuses 10 and 10' according to the present disclosure may calculate blood pressure in consideration of both the PTT and the ASI to more accurately measure the systolic blood pressure and the diastolic blood pressure.

Table 1 below shows an accuracy difference between the case in which blood pressure is estimated by applying an ASI like in the blood pressure measuring apparatuses 10 and 10' according to the present disclosure and the prior art in which blood pressure is estimated without application of an ASI.

Table 1

|  | Prior Art | Present disclosure |
|---|---|---|
| systolic blood pressure | ± 9.08 | ± 6.38 |
| diastolic blood pressure | ± 7.80 | ± 6.06 |

Referring to Table. 1 above, an accuracy difference in systolic blood pressure between the present disclosure and the prior art is 2.7 and the accuracy difference in diastolic blood pressure is 1.74. Accordingly, it may be seen that the accuracy according to the present disclosure is enhanced compared with the prior art by about 20% to 30%. As described above, with regard to the blood pressure measuring apparatuses 10 and 10' according to the present disclosure, when the pressure sensor 35' is wound around a portion of the wrist, if the blood pressure measuring apparatus 10' is separated from the wrist and, then, is re-worn on the wrist, a procedure of minutely adjusting a position of the wearing portion 20' to find a position of the radial artery as in the prior art may be omitted. In addition, an arterial stiffness index (ASI) is applied to calculate blood pressure and, thus, a separate structure or user effort for satisfying a similar wearing condition is not required every blood pressure measurement time point, thereby maximizing user convenience.

The blood pressure measuring apparatuses 10 and 10' according to the present disclosure may be small sized to be worn on the wrist like a wrist watch and, thus, it may be advantageous to conveniently managed and stored.

According to the present exemplary embodiment, to measure blood pressure, whether an appropriate hand shape is taken may be determined, and a user may be notified about the determination result to guide the user to take the appropriate hand shape.

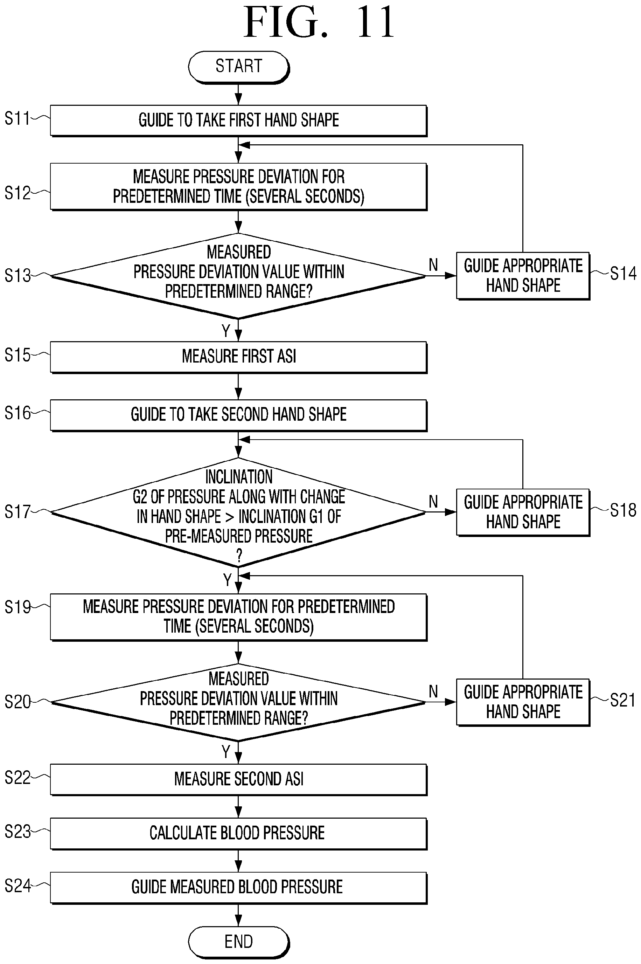

FIG. 11 is a flowchart showing a procedure of measuring blood pressure while guiding a user to take first and second hand shapes.

Hereinafter, the case in which the first hand shape to be described below is the hand shape H2 of FIG. 7A and the second hand shape is the hand shape H3 of FIG. 7A will be exemplified.

First, when a user begins to measure blood pressure by selecting a menu displayed on the display 50 of the blood pressure measuring apparatus 10 or pressing a predetermined command input button (not shown) included in the blood pressure measuring apparatus 10, an image corresponding to the first hand shape (the hand shape H2 of FIG. 7A) and/or a text for description of the image may be displayed through the display 50 to guide the user to take the corresponding hand shape (S11).

In this case, instead of the guidance through the display 50, guide speech may be output using voice through the micro speaker 70 to guide the first hand shape. In addition, through the display 50 and the micro speaker 70, an image and/or characters may be displayed and, simultaneously, guide speech may be output to guide the first hand shape.

The first hand shape may be guided and a pressure deviation value applied to the wrist for a predetermined time period (several seconds) is measured (S12) and, then, whether the measured pressure deviation value is within a predetermined range may be determined (S13).

When the measured pressure deviation value is not within the predetermined range, an appropriate hand shape with respect to the first hand shape may be guided to the user through the display 50 and/or the micro speaker 70 (S14). Operation S13 may be re-performed.

When the measured pressure deviation value is within the predetermined range, a first ASI may be measured (S15). The first ASI may be measured using the same method as the aforementioned ASI measuring method.

When the first ASI is completely measured, the user may be guided to take the second hand shape (the hand shape H3 of FIG. 7A) through the display 50 and/or the micro speaker 70 (S16).

Then, whether an inclination (i.e., an inclination of pressure applied to the wrist along with a posture change) of a portion G2 of the graph shown in FIG. 7A is greater than an inclination (i.e., an inclination of pressure applied to the wrist prior to a posture change) of a pre-measured portion G1 of the graph shown in FIG. 7A may be determined (S17). In this operation, whether the user changes a hand shape to the second hand shape from the first hand shape may be determined.

When the inclination of the portion G2 is less than the inclination of the pre-measured G1, the hand shape may not be determined to be appropriately changed to the second hand shape from the first hand shape and an appropriate hand shape with respect to the second hand shape may be guided to the user through the display 50 and/or the micro speaker 70 (S18). Operation S17 may be re-performed.

When the inclination of the portion G2 is greater than the inclination of the pre-measured G1, the hand shape may be determined to be appropriately changed to the second hand shape from the first hand shape and a pressure deviation value applied to the wrist for a predetermined time period (several seconds) (S19) and, then, whether the measured pressure deviation value is within the predetermined range may be determined (S20).

When the measured pressure deviation value is not within the predetermined range, an appropriate hand shape with respect to the second hand shape may be guided to the user through the display 50 and/or the micro speaker 70 (S21). Operation S19 may be re-performed.

When the measured pressure deviation value is within the predetermined range, a second ASI may be measured (S22). The second ASI may also be measured using the same method as the aforementioned ASI measuring method.

As such, when the first and second ASIs are measured, blood pressure may be calculated using the aforementioned method (S23) and the measured blood pressure may be guided to the user through the display 50 and/or the micro speaker 70 (S24).

As such, according to the present exemplary embodiment, to measure blood pressure, the user may be easily guided to take at least two different hand shapes, thereby more accurately measuring blood pressure.

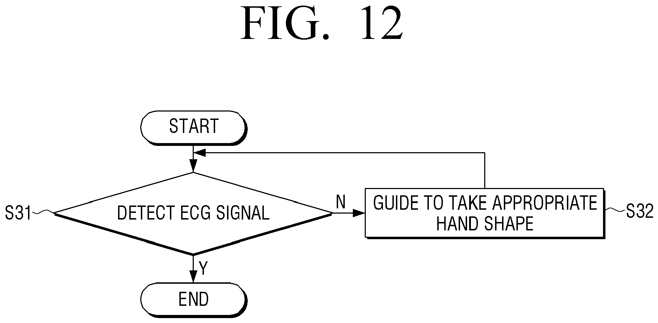

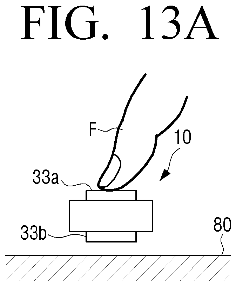

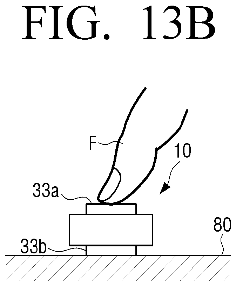

FIG. 12 is a flowchart showing a procedure of determining whether a wearing portion is appropriately worn on the wrist through an ECG signal. FIG. 13A is a diagram showing a state in which a second electrocardiogram electrode of an ECG sensor is spaced apart from the wrist. FIG. 13B is a diagram showing a state in which a second electrocardiogram electrode of an ECG sensor contacts the wrist.

When the blood pressure measuring apparatus 10 is worn on the wrist, if a wearing posture is not appropriate, a finger F contacts the first electrocardiogram electrode 33a of the ECG sensor 33 as shown in FIG. 13A, but the second electrocardiogram electrode 33b may be spaced apart from the wrist (in detail, an upper portion of the wrist) by a predetermined interval.

Under an appropriate wearing posture of the blood pressure measuring apparatus 10 for blood pressure measurement, the finger F may contact the first electrocardiogram electrode 33a of the ECG sensor 33 and the second electrocardiogram electrode 33b may contact the wrist (in detail, an upper portion of the wrist), as shown in FIG. 13B.

As such, when the second electrocardiogram electrode 33b of the ECG sensor 33 is spaced apart from the wrist, it may be difficult to accurately measure blood pressure and, thus, according to the present exemplary embodiment, a signal of the ECG sensor 33 may be detected to guide an appropriate wearing posture of the blood pressure measuring apparatus 10 to the user, as shown in FIG. 12. That is, during blood pressure measurement, whether the ECG signal is detected may be determined through the ECG sensor 33 (S31).

When the ECG signal is not detected, an appropriate wearing posture of the blood pressure measuring apparatus 10 may be guided to the user through the display 50 and/or the micro speaker 70 (S32). Operation S31 may be re-performed. When the ECG signal is detected, it may be determined that the blood pressure measuring apparatus 10 is appropriately worn and, continuously, blood pressure may be measured.

Industrial Applicability

The present isclosure relates to a blood pressure measuring apparatus and a blood pressure measuring method using the same.

Claims

1.   A blood pressure measuring method comprising:

   calculating, by a controller (60), a pulse transit time, PTT, using a photo-plethysmograph, PPG, signal and an electrocardiogram, ECG, signal measured by a sensor (30);
   calculating, by the controller (60), an arterial stiffness index, ASI; and
   calculating, by the controller (60), systolic blood pressure and diastolic blood pressure using the PTT and the ASI, wherein th ASI is calculated in each of at least two or more postures with different hand shapes by measuring, by the sensor (30), reference pressure applied to a wrist and fluctuation pressure according to cardiac impulse under the reference pressure with respect to each hand shape, obtaining, by the controller (60), a graph satisfying values corresponding to the reference pressure and the fluctuation pressure, and calculating, by the controller (60), a slope through two respectively consecutive points on the graph for which the reference pressure and the fluctuation pressure have been measured.

2.   The blood pressure measuring method as claimed in claim 1, wherein the hand shapes are taken by spreading all fingers or folding at least one finger.

3. The blood pressure measuring method as claimed in claim 1, wherein the hand shapes are taken by closing a fist or spreading at least one finger while the fist is closed.

4. The blood pressure measuring method as claimed in claim 1, wherein the hand shapes are taken by folding a finger.

5. The blood pressure measuring method as claimed in claim 1, wherein the hand shapes are taken by allowing at least two fingers to contact each other.

6. The blood pressure measuring method as claimed in claim 1, wherein the hand shapes are taken by spacing all fingers apart from each other.

7. The blood pressure measuring method as claimed in claim 1, wherein the hand shape is taken by bending the wrist in any one direction.

8. The blood pressure measuring method as claimed in claim 7, wherein the hand shape is taken by bending the wrist at a predetermined angle.

9. The blood pressure measuring method as claimed in claim 1, wherein the hand shape is taken by bending the wrist in any one direction.

10. The blood pressure measuring method as claimed in claim 1, further comprising:

   guiding a first hand shape using a display and/or a speaker;
   measuring a first pressure deviation applied to a wrist for a predetermined time;
   re-guiding the first hand shape when the first pressure deviation is not within a predetermined range and measuring a first ASI when the first pressure deviation is within the predetermined range;
   guiding a second hand shape using the display and/or the speaker;
   determining whether a first inclination of pressure applied to the wrist is greater than a second inclination of pre-measured pressure applied to the wrist as a hand shape is changed to the second hand shape from the first hand shape;
   re-guiding the second hand shape when the first inclination is less than the second inclination and measuring a second pressure deviation applied to the wrist for a predetermined time when the first inclination is greater than the second inclination; and
   re-guiding the second hand shape when the second pressure deviation is not within a predetermined range and measuring a second ASI when the second pressure deviation is within the predetermined range.

11. A blood pressure measuring apparatus (10) comprising:

   a wearing portion (20) configured to be worn on a wrist of a user;
   a sensor (30) disposed on the wearing portion (20) and configured to measure a photo-plethysmograph, PPG, signal and an electrocardiogram, ECG, signal,
   a controller (60) configured to calculate a pulse transit time, PTT, using the photo-plethysmograph, PPG, signal and the electrocardiogram, ECG, signal, and an arterial stiffness index, ASI, of the user and calculate systolic blood pressure and diastolic blood pressure of the user using the PTT and the ASI and
   a display configured to display the systolic blood pressure and diastolic blood pressure calculated by the controller (60),
   wherein the sensor (30) is further configured to
   detect pressure in a state in which at least two different hand shapes are taken to vary a degree pressurized to the wrist by the wearing portion (20),
   wherein the controller (60) is further configured to
   calculate the ASI by measuring, by the sensor (30), reference pressure applied to a wrist and fluctuation pressure according to cardiac impulse under the reference pressure with respect to each hand shape, obtain a graph satisfying values corresponding to the reference pressure and the fluctuation pressure, and calculate a slope through two respectively consecutive points on the graph for which the reference pressure and the fluctuation pressure have been measured.

**Patentansprüche**

1.  Verfahren zur Blutdruckmessung, umfassend:

    Berechnen, von einer Steuerung (60), einer Pulswellenlaufzeit, *pulse transit time* - PTT, unter Verwendung eines Photoplethysmograph-, PPG, Signals und eines Elektrokardiogramm-, EKG, Signals, die von einem Sensor (30) gemessen werden;
    Berechnen, von der Steuerung (60), eines Arteriensteifigkeitsindex, *arterial stiffness index* - ASI; und
    Berechnen, von der Steuerung (60), eines systolischen Blutdrucks und diastolischen Blutdrucks unter Verwendung der PTT und des ASI,
    wobei der ASI in jeder von mindestens zwei oder mehr Haltungen mit verschiedenen Handformen berechnet wird, durch Messen, von dem Sensor (30), eines Referenzdrucks, der an ein Handgelenk angelegt wird, und eines Fluktuationsdrucks gemäß einem Herzimpuls unter dem Referenzdruck in Bezug auf jede Handform, Erzeugen, von der Steuerung (60), eines Graphen, der Werte erfüllt, die dem Referenzdruck und dem Fluktuationsdruck entsprechen, und Berechnen, von der Steuerung (60), einer Steigung durch zwei jeweils aufeinanderfolgende Punkte auf dem Graphen, für die der Referenzdruck und der Fluktuationsdruck gemessen wurden.

2.  Verfahren zur Blutdruckmessung nach Anspruch 1, wobei die Handformen durch Spreizen aller Finger oder Beugen von mindestens einem Finger eingenommen werden.

3.  Verfahren zur Blutdruckmessung nach Anspruch 1, wobei die Handformen durch Ballen einer Faust oder Abspreizen von mindestens einem Finger während die Faust geballt ist, eingenommen werden.

4.  Verfahren zur Blutdruckmessung nach Anspruch 1, wobei die Handformen durch Beugen von einem Finger eingenommen werden.

5.  Verfahren zur Blutdruckmessung nach Anspruch 1, wobei die Handformen dadurch eingenommen werden, dass mindestens zwei Finger einander berühren dürfen.

6.  Verfahren zur Blutdruckmessung nach Anspruch 1, wobei die Handformen durch Beabstanden aller Finger voneinander eingenommen werden.

7.  Verfahren zur Blutdruckmessung nach Anspruch 1, wobei die Handform durch Biegen des Handgelenkes in eine beliebige Richtung eingenommen wird.

8.  Verfahren zur Blutdruckmessung nach Anspruch 7, wobei die Handform durch Biegen des Handgelenkes in einem vorbestimmten Winkel eingenommen wird.

9.  Verfahren zur Blutdruckmessung nach Anspruch 1, wobei die Handform durch Biegen des Handgelenkes in eine beliebige Richtung eingenommen wird.

10. Verfahren zur Blutdruckmessung nach Anspruch 1, ferner umfassend:

    Heranführen an eine erste Handform unter Verwendung einer Anzeige und/oder eines Lautsprechers;
    Messen einer ersten Druckabweichung, die für eine vorbestimmte Zeit an ein Handgelenk angelegt wird;
    erneutes Heranführen an die erste Handform, wenn die erste Druckabweichung nicht in einem vorbestimmten Bereich liegt, und Messen eines ersten ASI, wenn die erste Druckabweichung in dem vorbestimmten Bereich liegt;
    Heranführen an eine zweite Handform unter Verwendung der Anzeige und/oder des Lautsprechers;
    Bestimmen, ob eine erste Neigung von an das Handgelenk angelegtem Druck größer ist als eine zweite Neigung von an das Handgelenk angelegtem zuvor gemessenen Druck, während eine Handform von der ersten Handform zu der zweiten Handform geändert wird;
    erneutes Heranführen an die zweite Handform, wenn die erste Neigung geringer ist als die zweite Neigung, und Messen einer an das Handgelenk für eine vorbestimmte Zeit angelegten zweiten Druckabweichung, wenn die erste Neigung größer ist als die zweite Neigung; und
    erneutes Heranführen an die zweite Handform, wenn die zweite Druckabweichung nicht in einem vorbestimmten Bereich liegt, und Messen eines zweiten ASI, wenn die zweite Druckabweichung in dem vorbestimmten Bereich liegt.

**11.** Vorrichtung zur Blutdruckmessung (10) umfassend:

einen Trageabschnitt (20), der dazu ausgestaltet ist, an einem Handgelenk eines Benutzers getragen zu werden;
einen Sensor (30), der auf dem Trageabschnitt (20) angeordnet und dazu ausgestaltet ist, ein Photoplethysmograph-, PPG, Signal und ein Elektrokardiogramm-, EKG, Signal zu messen,
eine Steuerung (60), die dazu ausgestaltet ist, eine Pulswellenlaufzeit, *pulse transit time* - PTT, unter Verwendung des Photoplethysmograph-, PPG, Signals und des Elektrokardiogramm-, EKG, Signals, und einen Arteriensteifigkeitsindex, ASI, des Benutzers zu berechnen und einen systolischen Blutdruck und diastolischen Blutdruck des Benutzers unter Verwendung der PTT und des ASI zu berechnen, und
eine Anzeige, die dazu ausgestaltet ist, den von der Steuerung (60) berechneten systolischen Blutdruck und diastolischen Blutdruck anzuzeigen,
wobei der Sensor (30) ferner dazu ausgestaltet ist,
Druck durch den Trageabschnitt in einem Zustand zu erfassen, in dem mindestens zwei verschiedene Handformen eingenommen werden, um einen Grad des Drucks auf das Handgelenk, zu variieren,
wobei die Steuerung (60) ferner dazu ausgestaltet ist,
den ASI zu berechnen, durch Messen, von dem Sensor (30), eines an ein Handgelenk angelegten Referenzdrucks und Fluktuationsdrucks gemäß einem Herzimpuls unter dem Referenzdruck in Bezug auf jede Handform,
Erzeugen eines Graphen, der Werte erfüllt, die dem Referenzdruck und dem Fluktuationsdruck entsprechen, und Berechnen einer Steigung durch zwei jeweils aufeinanderfolgende Punkte auf dem Graphen, für die der Referenzdruck und der Fluktuationsdruck gemessen wurden.

## Revendications

**1.** Procédé de mesure de la tension artérielle, comprenant :

le calcul, par un dispositif de commande (60), d'un temps de transit du pouls, PTT, en utilisant un signal de photopléthysmographe, PPG, et un signal d'électrocardiogramme, ECG, mesurés par un capteur (30) ;
le calcul, par le dispositif de commande (60), d'un indice de rigidité artérielle, ASI ; et
le calcul, par le dispositif de commande (60), de la pression systolique et de la pression diastolique en utilisant le PTT et l'ASI,
dans lequel l'ASI est calculé dans chacune d'au moins deux postures ou plus, avec des formes de main différentes, en mesurant, par le capteur (30), la pression de référence appliquée à un poignet et la pression de fluctuation en fonction des impulsions cardiaques en dessous de la pression de référence par rapport à chaque forme de main, en obtenant, par le dispositif de commande (60), un graphique satisfaisant à des valeurs correspondant à la pression de référence et à la pression de fluctuation, et en calculant, par le dispositif de commande (60), une pente à travers deux points respectivement consécutifs sur le graphique, pour laquelle la pression de référence et la pression de fluctuation ont été mesurées.

**2.** Procédé de mesure de la tension artérielle selon la revendication 1, dans lequel les formes des mains sont prises en étendant tous les doigts ou en repliant au moins un doigt.

**3.** Procédé de mesure de la tension artérielle selon la revendication 1, dans lequel les formes des mains sont prises en fermant le poing ou en étendant au moins un doigt pendant que le poing est fermé.

**4.** Procédé de mesure de la tension artérielle selon la revendication 1, dans lequel les formes des mains sont prises en repliant un doigt.

**5.** Procédé de mesure de la tension artérielle selon la revendication 1, dans lequel les formes des mains sont prises en autorisant au moins deux doigts à venir en contact l'un avec l'autre.

**6.** Procédé de mesure de la tension artérielle selon la revendication 1, dans lequel les formes des mains sont prises en écartant tous les doigts à distance les uns des autres.

**7.** Procédé de mesure de la tension artérielle selon la revendication 1, dans lequel la forme de la main est prise en pliant le poignet dans une quelconque direction.

**8.** Procédé de mesure de la tension artérielle selon la revendication 7, dans lequel la forme de la main est prise en pliant le poignet à un angle prédéterminé.

**9.** Procédé de mesure de la tension artérielle selon la revendication 1, dans lequel la forme de la main est prise en pliant le poignet dans une quelconque direction.

**10.** Procédé de mesure de la tension artérielle selon la revendication 1, comprenant en outre :

le rapprochement d'une première forme de main en utilisant un affichage et/ou un haut-parleur ;
la mesure d'un premier écart de pression appliqué à un poignet pendant une durée prédéterminée ;
le re-rapprochement de la première forme de main lorsque le premier écart de pression n'est pas compris dans une plage prédéterminée et la mesure d'un premier ASI lorsque le premier écart de pression est compris dans la plage prédéterminée ;
le rapprochement d'une seconde forme de main en utilisant l'affichage et/ou le haut-parleur ;
la détermination si oui ou non une première inclinaison de pression appliquée au poignet est supérieure à une seconde inclinaison de pression pré-mesurée appliquée au poignet lorsqu'une forme de main est changée pour une seconde forme de main à partir de la première forme de main ;
le re-rapprochement de la seconde forme de main lorsque la première inclinaison est inférieure à la seconde inclinaison et la mesure d'un second écart de pression appliqué au poignet pendant une durée prédéterminée lorsque la première inclinaison est supérieure à la seconde inclinaison ; et
le re-rapprochement de la seconde forme de main lorsque le second écart de pression n'est pas compris dans une plage prédéterminée et la mesure d'un second ASI lorsque le second écart de pression est compris dans la plage prédéterminée.

**11.** Appareil de mesure de la tension artérielle (10), comprenant :

une portion à porter (20) configurée pour être portée sur le poignet d'un utilisateur ;
un capteur (30) disposé sur la portion à porter (20) et configuré pour mesurer un signal de photopléthysmographe, PPG, et un signal d'électrocardiogramme, ECG,
un dispositif de commande (60) configuré pour calculer un temps de transit du pouls, PTT, en utilisant le signal de photopléthysmographe, PPG, et le signal d'électrocardiogramme, ECG, et un indice de rigidité artérielle, ASI, de l'utilisateur et pour calculer la pression systolique et la pression diastolique de l'utilisateur en utilisant le PTT et l'ASI et
un affichage configuré pour afficher la pression systolique et la pression diastolique calculées par le dispositif de commande (60),
dans lequel le capteur (30) est en outre configuré pour
détecter la pression par la portion à porter dans un état où au moins deux formes de mains différentes sont prises pour faire varier un degré de pression au poignet,
dans lequel le dispositif de commande (60) est en outre configuré pour
calculer l'ASI en mesurant, par le capteur (30), la pression de référence appliquée à un poignet la pression de fluctuation en fonction des impulsions cardiaques en dessous de la pression de référence par rapport à chaque forme de main, obtenir un graphique satisfaisant à des valeurs correspondant à la pression de référence et à la pression de fluctuation, et calculer une pente à travers deux points respectivement consécutifs sur le graphique, pour laquelle la pression de référence et la pression de fluctuation ont été mesurées.

# FIG. 1

# FIG. 2A

# FIG. 2B

<u>10</u>

20

50

SBP 120
DBP 80

51

31

33a

47

43b

25

# FIG. 3

# FIG. 4

# FIG. 5

```
                    ┌──────────────┐
                    │    START     │
                    └──────┬───────┘
                           │
                           ▼
            ┌───────────────────────────────┐
            │   MEASURE INITIAL PRESSURE     │─── S1
            └───────────────┬───────────────┘
                            │
                            ▼
            ┌───────────────────────────────┐
            │          CALCULATE            │─── S2
            │        PTTo, bo, AND K        │
            └───────────────┬───────────────┘
                            │
        ┌───────────────────┤
        │                   ▼
        │   ┌───────────────────────────────┐
        │   │          CALCULATE            │─── S3
        │   │         PTT, b, AND K         │
        │   └───────────────┬───────────────┘
        │                   │
        │                   ▼
        │   ┌───────────────────────────────┐
        │   │   CALCULATE CURRENT PRESSURE   │─── S4
        │   └───────────────┬───────────────┘
        │                   │
        │                   ▼
        │   ┌───────────────────────────────┐
        │   │    DISPLAY CURRENT PRESSURE    │─── S5
        │   └───────────────┬───────────────┘
        │                   │
        └───────────────────┤
                            ▼
                    ┌──────────────┐
                    │     END      │
                    └──────────────┘
```

# FIG. 6

# FIG. 7A

# FIG. 7B

(a)

10

20

α1

(b)

10

20

α2

# FIG. 8

FLUCTUATION
PRESSURE

REFERENCE
PRESSURE

# FIG. 9

# FIG. 10

# FIG. 11

START

S11 — GUIDE TO TAKE FIRST HAND SHAPE

S12 — MEASURE PRESSURE DEVIATION FOR PREDETERMINED TIME (SEVERAL SECONDS)

S13 — MEASURED PRESSURE DEVIATION VALUE WITHIN PREDETERMINED RANGE? — N → GUIDE APPROPRIATE HAND SHAPE — S14

Y

S15 — MEASURE FIRST ASI

S16 — GUIDE TO TAKE SECOND HAND SHAPE

S17 — INCLINATION G2 OF PRESSURE ALONG WITH CHANGE IN HAND SHAPE > INCLINATION G1 OF PRE-MEASURED PRESSURE ? — N → GUIDE APPROPRIATE HAND SHAPE — S18

Y

S19 — MEASURE PRESSURE DEVIATION FOR PREDETERMINED TIME (SEVERAL SECONDS)

S20 — MEASURED PRESSURE DEVIATION VALUE WITHIN PREDETERMINED RANGE? — N → GUIDE APPROPRIATE HAND SHAPE — S21

Y

S22 — MEASURE SECOND ASI

S23 — CALCULATE BLOOD PRESSURE

S24 — GUIDE MEASURED BLOOD PRESSURE

END

# FIG. 12

START

S31 —— DETECT ECG SIGNAL —N→ GUIDE TO TAKE APPROPRIATE HAND SHAPE —— S32

Y

END

# FIG. 13A

# FIG. 13B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090018422 A1
- US 20100241011 A1
- US 20110009718 A1